Europäisches Patentamt

**European Patent Office**

Office européen des brevets

(11) Numéro de publication : **0 063 132
B1**

(12) **FASCICULE DE BREVET EUROPÉEN**

(45) Date de publication du fascicule du brevet :
30.07.86

(51) Int. Cl.⁴ : **A 61 K   9/52**, A 61 K 31/165

(21) Numéro de dépôt : 81902835.8

(22) Date de dépôt : 22.10.81

(86) Numéro de dépôt international :
**PCT/FR 81/00134**

(87) Numéro de publication internationale :
**WO/8201468 (13.05.82 Gazette 82/12)**

(54) **NOUVELLE FORME GALENIQUE D'ADMINISTRATION DU METOCLOPRAMIDE, SON PROCEDE DE PREPARATION ET MEDICAMENT COMPRENANT CETTE NOUVELLE FORME.**

(30) Priorité : 28.10.80 FR 8023028

(43) Date de publication de la demande :
27.10.82 Bulletin 82/43

(45) Mention de la délivrance du brevet :
30.07.86 Bulletin 86/31

(84) Etats contractants désignés :
DE GB LU NL SE

(56) Documents cités :
BE-A-   876 857
FR-A- 1 347 413
FR-A- 2 150 931
FR-A- 2 313 915
FR-A- 2 390 959
FR-A- 2 432 313
US-A- 2 963 402
Chemical Abstracts, volume 83, no. 14, publié le 6 octobre 1975, (Columbus, Ohio, US) R. Shekerdzhiiski et al.: "Antiemetic preparation in tablet form with depot action based on prolonged diffusion", voir page 427, colonne 2, abrégé no. 120770m
Le dossier contient des informations techniques présentées postérieurement au dépôt de la demande et ne figurant pas dans le présent fascicule.

(73) Titulaire : LARUELLE, Claude
Avenue Bellevue
F-06270 Villeneuve-Loubet (FR)

(72) Inventeur : LARUELLE, Claude
Avenue Bellevue
F-06270 Villeneuve-Loubet (FR)

(74) Mandataire : Ores, Irène et al
CABINET ORES 6, Avenue de Messine
F-75008 Paris (FR)

**0 063 132**

**Description**

La présente invention concerne une préparation galénique du métoclopramide, et le médicament comprenant cette préparation.

Le métoclopramide est connu depuis de nombreuses années. Sous la forme de dichlorhydrate ou de monochlorhydrate, il est largement utilisé pour traiter les troubles fonctionnels gastro-intestinaux tels que dyspepsies, dyskinésies gastro-duodénales et biliaires, gastrites chroniques, ulcères gastro-duodénaux, nausées et vomissements. Au plan pharmacologique, l'action du métoclopramide est à la fois centrale et périphérique.

Toutefois, l'administration du métoclopramide sous sa forme actuelle présente des inconvénients notables en limitant la commodité d'emploi.

Par exemple, on a constaté que la présentation sous forme de comprimés provoque certaines intolérances, en particulier gastriques, ce qui empêche son utilisation par une catégorie importante de malades d'une part, et d'autre part qui limite son utilisation pour les traitements à long terme.

De plus, on a constaté avec les formes disponibles, après chaque administration, c'est-à-dire trois ou quatre fois par jour sinon davantage, une succession d'augmentations et de diminutions rapides des taux plasmatiques, l'organisme étant soumis alternativement à des surdosages et à des sous-dosages.

Enfin, les inconvénients des formes classiques n'ont pas permis jusqu'à présent le traitement des troubles digestifs nécessitant une administration prolongée, ce qui est au contraire possible avec la nouvelle forme galénique d'administration du métoclopramide ainsi qu'on le démontre ci-après, cette nouvelle forme permettant la libération régulière du médicament dans l'organisme pendant des périodes suffisamment longues pour autoriser simplement la prise journalière d'une seule unité.

La présente invention a donc pour objet une forme galénique à libération programmée retardée du métoclopramide et est caractérisée en ce qu'elle contient 20 mg de métoclopramide et en ce qu'elle est constituée par des microgranules comprenant une âme neutre constituée d'un grain d'un excipient inerte comprenant de 40 à 80 % en poids de saccharose et de 10 à 40 % en poids d'amidon, ce grain neutre étant muni d'une première couche comprenant de 1 à 20 % en poids de métoclopramide, de 0,01 % à 0,5 % en poids d'acide stéarique, de 5 à 15 % en poids de talc puis d'une seconde couche extérieure constituée par une enveloppe microporeuse comprenant au moins un polymère naturel et/ou synthétique appartenant à la classe constituée par la gomme laque, selon une proportion en poids comprise entre 1 et 10 %, la gomme arabique, la gélatine, l'éthylcellulose, l'acétophtalate de cellulose, le triacétate de cellulose, le polyoxyéthylèneglycol, les méthacrylates, le copolymère styrène-acrylonitrile et le polyvinylpyrrolidone en enveloppes successives.

En outre, l'âme neutre peut comprendre des adsorbats de métoclopramide.

L'invention a également pour objet un procédé d'obtention de forme galénique remarquable en ce qu'on prépare au préalable des microgranules neutres tamisés et séchés, qu'on projette sur ces microgranules une solution dans l'alcool absolu de métoclopramide, qu'on enrobe ensuite lesdits microgranules pour réaliser la première couche en un ou plusieurs enrobages, puis qu'on forme l'enveloppe microporeuse par enrobage à l'aide dudit polymère en solution dans un solvant.

De plus, la présente invention a pour objet des médicaments comprenant la forme d'administration du métoclopramide sous forme de microgranules actifs mélangés à des microgranules neutres non enrobés afin d'obtenir une concentration prédéterminée en métoclopramide, cet ensemble de microgranules étant présenté sous formes de gélules, de comprimés, de suppositoires, de sirop, de granules ou de poudre.

L'ensemble des caractéristiques et avantages de l'invention seront mieux compris par l'Homme de l'Art en se référant à la description qui va suivre de modes de réalisation particuliers pris à titre d'exemples non limitatifs de la nouvelle forme galénique, de son procédé et de ses applications thérapeutiques, en particulier en relation avec les contrôles pharmacologiques et cliniques effectués en utilisant la forme galénique.

Exemple de préparation de la forme galénique :

On indique ci-après, l'exemple de fabrication correspondant à 100 000 gélules dosées à 20 mg de chlorhydrate de métoclopramide.

a) Formule de fabrication :

— métoclopramide (dichlorhydrate monohydrate)                                                    2,0 kg
— saccharose
— amidon de maïs
— acide stéarique
— gomme laque
— polymères méthacryliques
— talc

2

0 063 132

— polyvidone
— édétate de sodium
— alcool éthylique absolu                                              qs 21,0 kg

b) Procédé de préparation

On granule de l'amidon de maïs et du saccharose, puis on tamise et on turbine longuement les grains de façon à les rendre parfaitement sphériques ; on tamise à nouveau et on sèche parfaitement. Dans un mélangeur en acier inoxydable, on projette sur les âmes neutres ainsi obtenues, une solution alcoolique de chlorhydrate de métoclopramide. On réalise ensuite la première couche en incorporant à ces microgranules les autres excipients à l'exception de la gomme laque, puis on recommence la pulvérisation de chlorhydrate de métoclopramide, cet enrobage étant recommencé plusieurs fois avec tamisage et séchage si nécessaire entre chaque couche.

Lorsque la première couche contenant le principe actif est terminée, on réalise la couche extérieure microporeuse en projetant sur les granules la gomme laque en solution dans l'alcool éthylique absolu.

On sèche ensuite soigneusement en éliminant l'alcool éthylique restant, on tamise à nouveau et on contrôle comme ci-après le titre des microgranules obtenus avant de mettre en gélules après avoir ajusté éventuellement le titrage par addition et homogénéisé avec des microgranules neutres pour arriver au titrage désiré de 20 mg de métoclopramide.

Mesure de la libération du métoclopramide

L'enveloppe extérieure microporeuse est réalisée de manière à permettre une libération prolongée de métoclopramide théorique :
1ère heure : libération inférieure à 40 % ;
4ème heure : libération inférieure à 75 % ;
8ème heure : libération supérieure à 80 %.

Pour contrôler cette caractéristique, on utilise un appareil à délitement dans lequel on met en contact une quantité de microgranules correspondant à environ 50 mg de principe actif avec des liquides artificiels, l'appareil permettant de maintenir une agitation constante et une température constante de $37° \pm 0,5$ °C. Les liquides artificiels sont des solutions tamponnées à pH successifs utilisés selon le schéma ci-dessous.

| SOLUTIONS | TEMPS DE LIBERATION | pH | RESULTATS | |
| --- | --- | --- | --- | --- |
| | | | THEORIQUES | REELS |
| 25 ml liquide gastrique | 1 heure (1ère heure) | 1,5 | < 40 % | 36 % |
| 25 ml liquide intestinal | 1 heure (2ème heure) | 4,5 | > 40 % | |
| 25 ml liquide intestinal | 2 heures (3ème et 4ème heures) | 6,9 | < 75 % | 70,8 % |
| 25 ml liquide intestinal | 2 heures (5ème et 6ème heures) | 6,9 | > 75 % | |
| 25 ml liquide intestinal | 2 heures (7ème et 8ème heures) | 7,2 | > 80 % | 92,3 % |

La forme galénique selon l'invention a fait l'objet d'une étude pharmacocinétique approfondie en comparaison avec la forme comprimé classique. L'étude a été effectuée en cross-over chez l'homme. Six sujets de sexe masculin recevaient chacune des deux formes à deux semaines d'intervalle, une gélule de 20 mg de principe actif contenant les microgranules et 2 comprimés à 10 mg de la forme classique.

La détermination de la concentration plasmatique du métoclopramide a été effectuée au moyen de 10 prélèvements dans l'intervalle de 72 heures.

Au terme de l'étude, les conclusions sont les suivantes :
— La biodisponibilité relative n'est pas modifiée de manière significative.
— Le temps d'apparition du pic sérique passe de 1 heure à 4 heures et demie.
— La durée de demi-vie passe de 3 heures environ à plus de 8 heures.
— Les deux formulations sont bio-inéquivalentes.
— L'étude des courbes obtenues montre qu'une gélule à 20 mg équivaut à 2 comprimés au minimum, sinon 3 comprimés à 10 mg.

Afin d'illustrer les caractéristiques de l'invention rapportée ci-dessus, 3 graphiques sont présentés.

En outre, l'étude toxicologique effectuée sur le rat a permis de déterminer la dose léthale 50 % lorsque les microgranules sont administrés par voie orale :
— chez les femelles, la mortalité est de 50 % à 9,2 g/kg ;
— chez les mâles, la mortalité est de 40 % à 20 g/kg.

Au plan clinique, la tolérance de la nouvelle présentation a été bonne dans l'ensemble et supérieure à la présentation classique. Elle permet ainsi de pratiquer des traitements de longue durée sans inconvénients notable pour le patient.

De plus, du fait de l'économie de 35 % à 50 % en dosage de principe actif, on obtient donc une utilisation thérapeutique améliorée justifiant l'intérêt de la nouvelle présentation galénique.

Par conséquent, on peut affirmer que la nouvelle présentation galénique conduit à un nouveau médicament utile pour le traitement des troubles gastriques en général.

Elle permet d'assurer une libération du métoclopramide de façon contrôlée et indépendamment de la façon dont les microgranules sont administrés avec une courbe de libération du principe actif constante d'un malade à l'autre et d'une prise à l'autre.

Enfin, elle permet une mise en gélules et assure la stabilité du métoclopramide, ce qui est une amélioration industrielle et médicale importante.

Bien entendu, l'Homme de l'Art pourra trouver d'autres avantages et variantes de l'invention, en particulier en ce qui concerne le procédé d'obtention des microgranules ou les modifications de dosage, sans pour cela sortir du cadre et de la portée de la présente invention.

Explications des figures

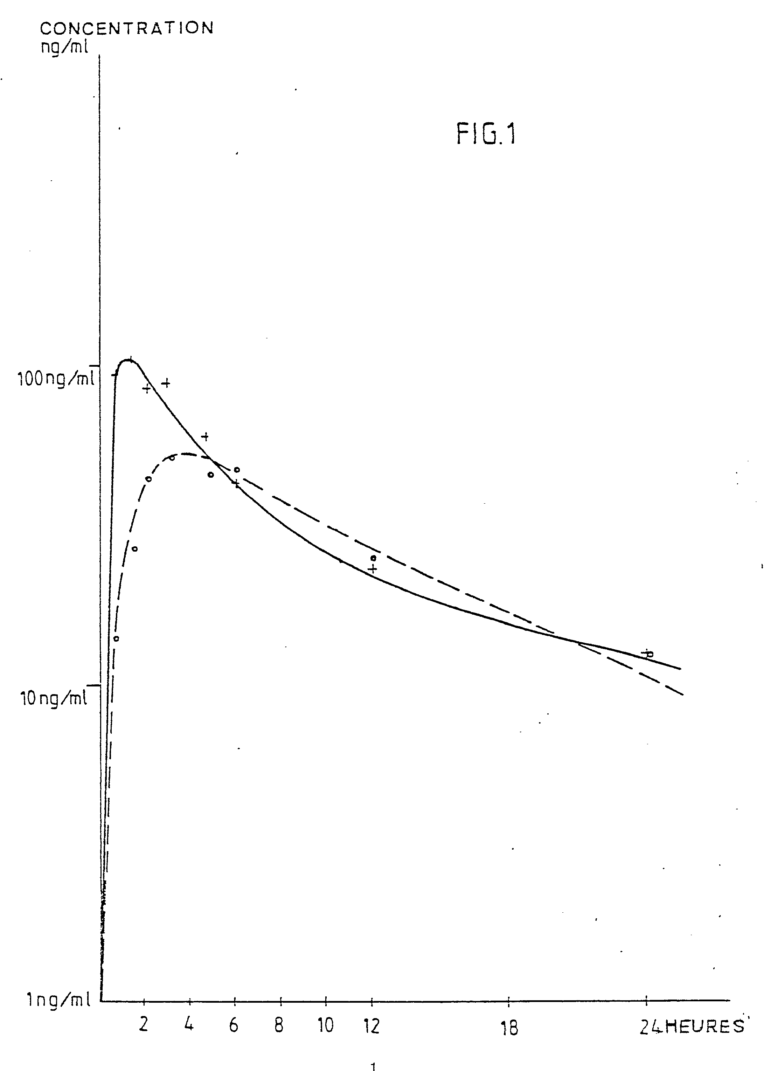

Figure 1 Concentrations plasmatiques de métoclopramide après une prise orale effectuée en cross-over chez 6 sujets sains
— 2 comprimés à 10 mg (———)
— 1 gélule retard à 20 mg (-----)

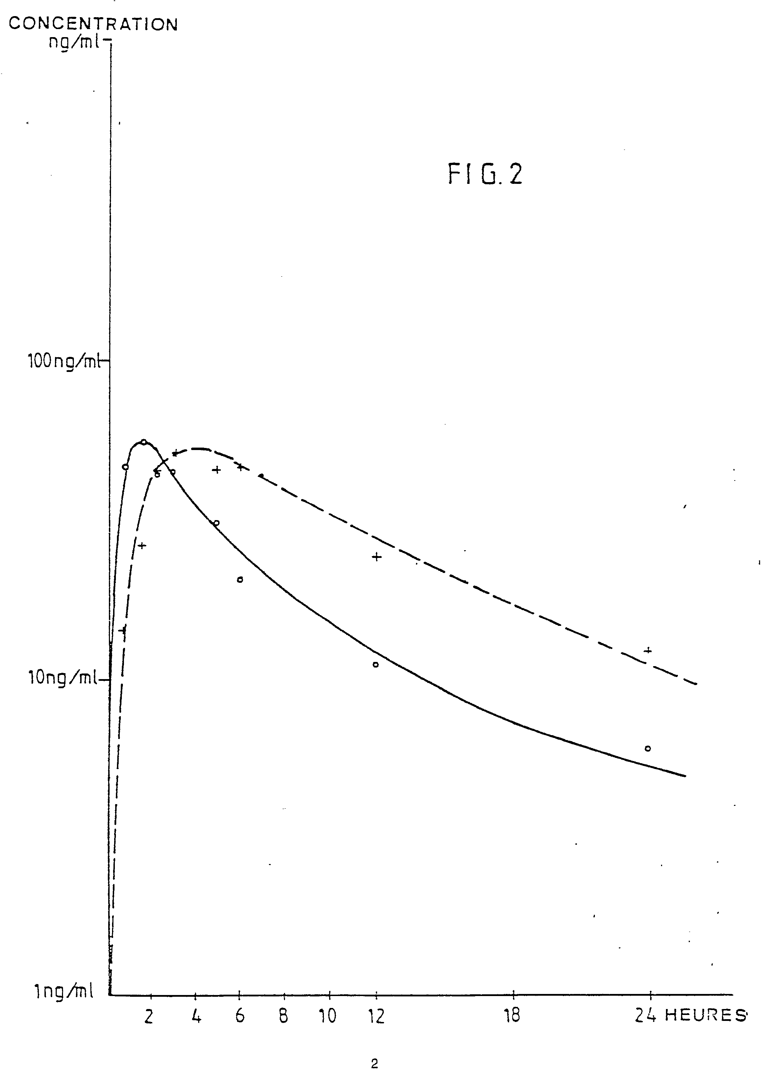

Figure 2 Concentrations plasmatiques de métoclopramide après une prise orale effectuée en cross-over chez 6 sujets sains
— gélule retard à 20 mg (-----)
— comprimé à 10 mg (———)

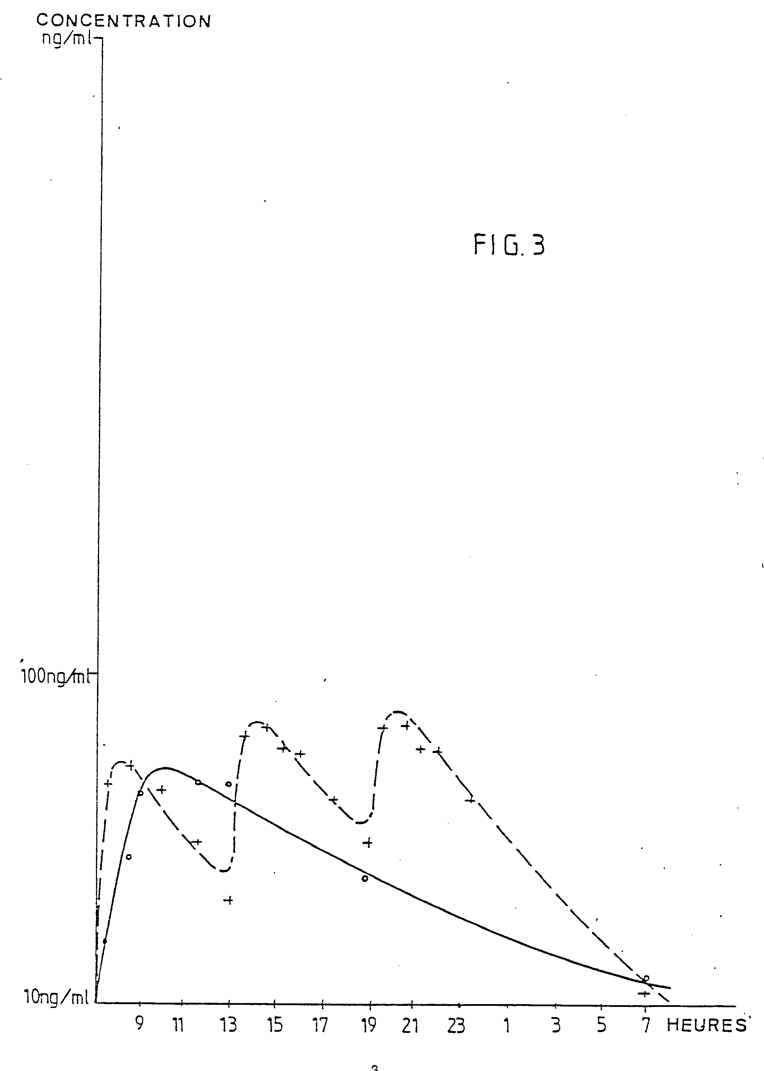

Figure 3 Concentrations plasmatiques de métoclopramide
— après trois prises orales à 7 H, 13 H, 19 H d'un comprimé à 10 mg (---- )
— après une prise orale à 7 H d'une gélule retard à 20 mg (———)

**Revendications**

1. Forme galénique du métoclopramide à libération prolongée dans le temps, caractérisée en ce qu'elle contient 20 mg de métoclopramide et en ce qu'elle est constituée par des micro-granules comprenant une âme neutre constituée d'un grain d'un excipient inerte constitué d'un mélange comprenant de 40 à 80 % en poids de saccharose et de 10 à 40 % en poids d'amidon, ce grain neutre étant muni d'une première couche constituée de 1 à 20 % en poids de métoclopramide, de 0,01 à 0,5 % en poids d'acide stéarique et de 5 à 15 % en poids de talc, puis d'une seconde couche extérieure constituée par une enveloppe microporeuse comprenant au moins un polymère naturel et/ou synthétique appartenant à la classe constituée par la gomme laque, selon une proportion en poids comprise entre 1 et 10 %, la gomme arabique, la gélatine, l'éthylcellulose, l'acétophtalate de cellulose, le triacétate de cellulose, le polyoxyéthylèneglycol, les méthacrylates, le copolymère styrène-acrylonitrile et la polyvinylpyrrolidone, et ce ce que lesdits microgranules sont mélangés avec des microgranules neutres non enrobés, afin d'obtenir une concentration prédéterminée en métoclopramide.

2. Forme selon la revendication 1, caractérisée en ce que l'âme neutre comprend des adsorbats de métoclopramide.

3. Procédé d'obtention de la forme galénique selon l'une quelconque des revendications 1 et 2, caractérisé en ce qu'on prépare des microgranules neutres tamisés et séchés, qu'on projette sur ces microgranules neutres une solution dans l'alcool absolu de métoclopramide sous forme de microgranules conformes à l'une quelconque des revendications 1 et 2, et en ce que lesdits microgranules sont mélangés avec des microgranules neutres non enrobés afin d'obtenir une concentration prédéterminée en métoclopramide.

4. Médicaments caractérisés en ce qu'ils comprennent la forme galénique d'administration du métoclopramide sous forme de microgranules conformes à l'une quelconque des revendications 1 et 2, et en ce que lesdits microgranules sont mélangés avec des microgranules neutres non enrobés afin d'obtenir une concentration pré-déterminée en métoclopramide.

**Claims**

1. Galenic form of metoclopramide with prolonged release over time, characterized in that it contains 20 mg of metoclopramide and in that it is constituted by microgranules comprising a neutral core constituted by a grain of an inert excipient constituted by a mixture comprising from 40 to 80 % by weight of saccharose and from 10 to 40 % by weight of starch, this neutral grain being provided with a first layer constituted by 1 to 20 % by weight of metoclopramide, from 0.01 to 0.5 % by weight of stearic acid and from 5 to 15 % by weight of talc, then a second outer layer constituted by a microporous envelope comprising at least one natural and/or synthetic polymer belonging to class constituted by gum lac, in a proportion by weight comprised between 1 and 10 %, gum arabic, gelatin, ethylcellulose, cellulose acetophthalate, cellulose triacetate, polyoxyethylene-glycol, methacrylates, styrene-acrylonitrile copolymer and polyvinylpyrrolidone, and in that said microgranules are mixed with uncoated neutral microgranules, in order to obtain a predetermined concentration of metoclopramide.

2. Form according to Claim 1, characterized in that the neutral core comprises metoclopramide adsorbates.

3. Process for obtaining the galenic form according to any one of Claims 1 and 2, characterized in that sifted and dried neutral microgranules are prepared, on the neutral microgranules is projected a solution in absolute alcohol of metoclopramide in the form of microgranules according to any one of Claims 1 and 2, and in that said microgranules are mixed with uncoated neutral microgranules in order to obtain a predetermined concentration of metoclopramide.

4. Medicaments characterized in that they comprise the galenic administrative form of metoclopramide in the form of microgranules according to any one of Claims 1 and 2, and in that said microgranules are mixed with uncoated neutral microgranules in order to obtain a predetermined concentration of metoclopramide.

**Patentansprüche**

1. Galenische Form des Metoclopramids mit zeitlich verlängerter Freisetzung, dadurch gekennzeichnet, daß sie 20 mg Metoclopramid enthält und daß sie von Mikrokörnchen gebildet ist, die eine neutrale Seele enthalten, welche von einem Körnchen eines inerten Bindemittels gebildet ist, das von einer Mischung gebildet ist, die von 40 bis 80 Gew.-% Saccharose und von 10 bis 40 Gew.-% Stärke enthält, wobei dieses neutrale Körnchen mit einem ersten Überzug versehen ist, der von 1 bis 20 Gew.-% Metoclopramid, von 0,01 bis 0,5 Gew.-% Stearinsäure und von 5 bis 15 Gew.-% Talkum gebildet ist, sowie danach mit einem zweiten äußeren Überzug, der aus einer mikroporösen Hülle besteht, die wenigstens ein natürliches und/oder synthetisches Polymer enthält, das zu der Klasse gehört, die gebildet ist von Schellack, nach einem Gewichtsverhältnis, das zwischen 1 und 10 % umfaßt, Gummi Arabikum, Gelatine, Ethylcellulose, Celluloseacetophtalat, Cellulosetriacetat, Polyoxyethylenglycol, Methacrylate, Styrol-Acrylnitril-Copolymer und Polyvinylpyrrolidon, und dadurch, daß diese Mikrokörnchen mit neutralen Mikrokörnchen, die nicht mit einer Hülle versehen sind, gemischt sind, um eine vorbestimmte Konzentration an Metoclopramid zu erhalten.

2. Form nach Anspruch 1, dadurch gekennzeichnet, daß die neutrale Seele Adsorbate von Metoclopramid enthält.

3. Verfahren zur Herstellung der galenischen Form gemäß einem der Ansprüche 1 und 2, dadurch gekennzeichnet, daß man ausgesiebte und getrocknete neutrale Mikrokörnchen herstellt, daß man auf diese neutralen Mikrokörnchen eine Lösung von Metoclopramid in der Form der Mikrokörnchen gemäß einem der Ansprüche 1 und 2 in absolutem Alkohol sprüht, und daß diese Mikrokörnchen mit neutralen Mikrokörnchen, die nicht mit einer Umhüllung versehen sind, gemischt werden, um eine vorbestimmte Konzentration an Metoclopramid zu erhalten.

4. Arzneimittel, dadurch gekennzeichnet, daß es die galenische Verabreichungsform des Metoclopramids in der Form von Mikrokörnchen gemäß einem der Ansprüche 1 und 2 enthält, und dadurch, daß diese Mikrokörnchen mit neutralen Mikrokörnchen, die nicht mit einer Umhüllung versehen sind, gemischt sind, um eine vorbestimmte Konzentration an Metoclopramid zu erhalten.

FIG.1

FIG. 2

0 063 132

FIG. 3